# EUROPEAN PATENT APPLICATION

(11) **EP 3 909 968 A1**
(43) Date of publication of application: **17.11.2021**
(21) Application number: 19906739.8
(22) Date of filing: 27.12.2019
(51) Int. Cl.: C07K 7/06, C07K 7/08, C07K 14/62

(54) **RAMP TAG FOR INSULIN OVEREXPRESSION AND METHOD FOR MANUFACTURING INSULIN USING SAME**

(30) Priority: 31.12.2018 KR 20180173365
(71) Applicant: Polus Inc., Incheon 21984 (KR)
(72) Inventor: KIM, Jinhwan, Incheon 21988 (KR); CHOI, Hyoung An, Incheon 21510 (KR); LEE, Sang Yeon, Incheon 21568 (KR)
(74) Representative: Budde Schou A/S
(86) International application number: PCT/KR2019/018548
(87) International publication number: WO 2020/141805

(57) **Abstract**

The present invention relates to a ramp tag for insulin overexpression represented by an amino acid sequence of RGSX₁GGX₂R (wherein, X₁ means any amino acids in the number of 0-8, and X₂ means S or T) and a method for manufacturing insulin, in which the ramp tag is applied, and when the ramp tag according to the present invention is used, in the process of manufacturing recombinant proteins, without changing the ORF sequence, such as the codon optimization method, translation efficiency of insulin is increased, thereby providing the effect of remarkably increasing the expression amount of insulin.

<110>    POLUS Inc.

<120>    Ramp Tag for Overexpressing Insulin and Method for Producing
         Insulin Using the Same

<130>    PP-B2349

<150>    KR 10-2018-0173365
<151>    2018-12-31

<160>    104

<170>    KoPatentIn 3.0

<210>    1
<211>    8
<212>    PRT
<213>    Artificial Sequence

<220>
<223>    Ramp1


<400>    1
Arg Leu Ala Pro Phe Cys Lys Thr
  1               5


<210>    2
<211>    8
<212>    PRT
<213>    Artificial Sequence

<220>
<223>    Ramp2


<400>    2
Phe His Leu Leu Arg Pro Lys Cys
  1               5


<210>    3
<211>    8
<212>    PRT
<213>    Artificial Sequence

<220>
<223>    Ramp3


<400>    3
Arg Thr Phe Leu Phe Leu Leu Cys
  1               5


<210>    4
<211>    8
<212>    PRT
<213>    Artificial Sequence

<220>
<223>    Ramp4

<400>    4
Arg His Lys Leu Leu Pro Phe Cys
1               5

<210>    5
<211>    15
<212>    PRT
<213>    Artificial Sequence

<220>
<223>    Ramp5

<400>    5
Arg Gly Ser Arg Cys Arg Cys Phe Thr Phe Thr Gly Gly Ser Arg
1               5                   10                  15

<210>    6
<211>    8
<212>    PRT
<213>    Artificial Sequence

<220>
<223>    Ramp6

<400>    6
Phe Cys Arg Thr Arg Thr Leu Leu
1               5

<210>    7
<211>    8
<212>    PRT
<213>    Artificial Sequence

<220>
<223>    Ramp7

<400>    7
Arg Pro Phe Cys Ala Thr Leu Leu
1               5

<210>    8
<211>    8
<212>    PRT
<213>    Artificial Sequence

<220>
<223>    Ramp8

<400>    8
Ala Thr Phe Leu Arg Pro Lys His
1               5

<210>    9
<211>    15
<212>    PRT
<213>    Artificial Sequence

<220>
<223>    Ramp9


<400>    9
Arg Gly Ser Leu Leu Arg Cys Arg Cys Phe Thr Gly Gly Ser Arg
1               5                   10                  15


<210>    10
<211>    8
<212>    PRT
<213>    Artificial Sequence

<220>
<223>    Ramp10


<400>    10
Arg Pro Ala Leu Phe Cys Lys His
1               5


<210>    11
<211>    8
<212>    PRT
<213>    Artificial Sequence

<220>
<223>    Ramp11


<400>    11
Arg Leu Phe Thr Phe Thr Leu Cys
1               5


<210>    12
<211>    15
<212>    PRT
<213>    Artificial Sequence

<220>
<223>    Ramp12


<400>    12
Arg Gly Ser Leu Leu Leu Leu Arg Thr Arg Thr Gly Gly Ser Arg
1               5                   10                  15


<210>    13
<211>    8
<212>    PRT
<213>    Artificial Sequence

<220>
<223>    Ramp13

<400>    13
Leu His Arg Leu Ala Pro Phe Cys
1               5

<210>    14
<211>    8
<212>    PRT
<213>    Artificial Sequence

<220>
<223>    Ramp14

<400>    14
Phe Thr Phe Thr Leu Leu Leu Leu
1               5

<210>    15
<211>    8
<212>    PRT
<213>    Artificial Sequence

<220>
<223>    Ramp15

<400>    15
Arg Leu Arg Leu Leu Thr Leu Thr
1               5

<210>    16
<211>    8
<212>    PRT
<213>    Artificial Sequence

<220>
<223>    Ramp16

<400>    16
Lys His Phe Cys Arg Pro Ala Thr
1               5

<210>    17
<211>    8
<212>    PRT
<213>    Artificial Sequence

<220>
<223>    Ramp17

<400>    17
Arg Cys Phe Thr Phe Thr Leu Leu
1               5

<210>    18
<211>    8
<212>    PRT
<213>    Artificial Sequence

<220>
<223>    Ramp18


<400>    18
Leu Thr Leu Thr Arg Leu Arg Leu
1               5


<210>    19
<211>    15
<212>    PRT
<213>    Artificial Sequence

<220>
<223>    Ramp19


<400>    19
Arg Gly Ser Phe Thr Leu Leu Leu Leu Arg Cys Gly Gly Ser Arg
1               5                   10                  15


<210>    20
<211>    8
<212>    PRT
<213>    Artificial Sequence

<220>
<223>    Ramp20


<400>    20
Phe Thr Phe Thr Arg Leu Arg Leu
1               5


<210>    21
<211>    8
<212>    PRT
<213>    Artificial Sequence

<220>
<223>    Ramp21


<400>    21
Leu Leu Arg Arg Arg Arg Phe Thr
1               5


<210>    22
<211>    8
<212>    PRT
<213>    Artificial Sequence

<220>
<223>    Ramp22

<400>    22
Leu Leu Arg Ile Arg Arg Phe Thr
1               5

<210>    23
<211>    8
<212>    PRT
<213>    Artificial Sequence

<220>
<223>    Ramp23

<400>    23
Leu Leu Arg Arg Arg Ile Phe Thr
1               5

<210>    24
<211>    8
<212>    PRT
<213>    Artificial Sequence

<220>
<223>    RampA

<400>    24
Arg Gly Ser Ala Gly Gly Ser Arg
1               5

<210>    25
<211>    8
<212>    PRT
<213>    Artificial Sequence

<220>
<223>    RampD

<400>    25
Arg Gly Ser Asp Gly Gly Ser Arg
1               5

<210>    26
<211>    8
<212>    PRT
<213>    Artificial Sequence

<220>
<223>    RampG

<400>    26
Arg Gly Ser Gly Gly Gly Ser Arg
1               5

<210>    27
<211>    7
<212>    PRT
<213>    Artificial Sequence

<220>
<223>    RampH


<400>    27
Arg Gly Ser Gly Gly Ser Arg
1               5


<210>    28
<211>    15
<212>    PRT
<213>    Artificial Sequence

<220>
<223>    Rampi


<400>    28
Arg Gly Ser Leu Leu Arg Ile Arg Ile Phe Thr Gly Gly Ser Arg
1               5                   10                  15


<210>    29
<211>    8
<212>    PRT
<213>    Artificial Sequence

<220>
<223>    RampK


<400>    29
Arg Gly Ser Lys Gly Gly Ser Arg
1               5


<210>    30
<211>    8
<212>    PRT
<213>    Artificial Sequence

<220>
<223>    RampN


<400>    30
Arg Gly Ser Asn Gly Gly Ser Arg
1               5


<210>    31
<211>    8
<212>    PRT
<213>    Artificial Sequence

<220>
<223>    RampR

<400>    31
Arg Gly Ser Arg Gly Gly Ser Arg
1               5


<210>    32
<211>    24
<212>    DNA
<213>    Artificial Sequence

<220>
<223>    Ramp1


<400>    32
cgcctagccc ctttttgtaa aaca                                              24


<210>    33
<211>    24
<212>    DNA
<213>    Artificial Sequence

<220>
<223>    Ramp2


<400>    33
tttcacttgc tacgccctaa atgt                                              24


<210>    34
<211>    24
<212>    DNA
<213>    Artificial Sequence

<220>
<223>    Ramp3


<400>    34
cgcacatttc tatttctatt gtgt                                              24


<210>    35
<211>    24
<212>    DNA
<213>    Artificial Sequence

<220>
<223>    Ramp4


<400>    35
cgccacaaac tattgccttt ttgt                                              24


<210>    36
<211>    45
<212>    DNA
<213>    Artificial Sequence

<220>
<223>    Ramp5

<400>    36
agaggatcac gctgtcgctg ttttacattt acaggaggat cacga                45

<210>    37
<211>    24
<212>    DNA
<213>    Artificial Sequence

<220>
<223>    Ramp6

<400>    37
ttttgtcgca cacgcacatt gcta                                       24

<210>    38
<211>    24
<212>    DNA
<213>    Artificial Sequence

<220>
<223>    Ramp7

<400>    38
cgcccttttt gtgccacatt gcta                                       24

<210>    39
<211>    24
<212>    DNA
<213>    Artificial Sequence

<220>
<223>    Ramp8

<400>    39
gccacatttc tacgccctaa acac                                       24

<210>    40
<211>    45
<212>    DNA
<213>    Artificial Sequence

<220>
<223>    Ramp9

<400>    40
agaggatcat tgctacgctg tcgctgtttt acaggaggat cacga                45

<210>    41
<211>    24

<212>    DNA
<213>    Artificial Sequence

<220>
<223>    Ramp10


<400>    41
cgccctgccc tattttgtaa acac                                              24


<210>    42
<211>    24
<212>    DNA
<213>    Artificial Sequence

<220>
<223>    Ramp11


<400>    42
cgcctattta catttacatt gtgt                                              24


<210>    43
<211>    63
<212>    DNA
<213>    Artificial Sequence

<220>
<223>    Ramp12


<400>    43
agaggatcac atcaccatca ccatcacttg ctattgctac gcacacgcac aggaggatca      60

cga                                                                     63


<210>    44
<211>    24
<212>    DNA
<213>    Artificial Sequence

<220>
<223>    Ramp13


<400>    44
ttgcaccgcc tagccccttt ttgt                                              24


<210>    45
<211>    24
<212>    DNA
<213>    Artificial Sequence

<220>
<223>    Ramp14


<400>    45
tttacattta cattgctatt gcta                                              24

<210>    46
<211>    24
<212>    DNA
<213>    Artificial Sequence

<220>
<223>    Ramp15


<400>    46
cgcctacgcc tattgacatt gaca                                              24


<210>    47
<211>    24
<212>    DNA
<213>    Artificial Sequence

<220>
<223>    Ramp16


<400>    47
aaacactttt gtcgccctgc caca                                              24


<210>    48
<211>    24
<212>    DNA
<213>    Artificial Sequence

<220>
<223>    Ramp17


<400>    48
cgctgtttta catttacatt gcta                                              24


<210>    49
<211>    24
<212>    DNA
<213>    Artificial Sequence

<220>
<223>    Ramp18


<400>    49
ttgacattga cacgcctacg ccta                                              24


<210>    50
<211>    63
<212>    DNA
<213>    Artificial Sequence

<220>
<223>    Ramp19

<400>    50
agaggatcac atcaccatca ccatcacttt acattgctat tgctacgctg tggaggatca    60

cga                                                                   63


<210>    51
<211>    24
<212>    DNA
<213>    Artificial Sequence

<220>
<223>    Ramp20


<400>    51
tttacattta cacgcctacg ccta                                            24


<210>    52
<211>    24
<212>    DNA
<213>    Artificial Sequence

<220>
<223>    Ramp21


<400>    52
ttgctacgcc gacgccgatt taca                                            24


<210>    53
<211>    24
<212>    DNA
<213>    Artificial Sequence

<220>
<223>    Ramp22


<400>    53
ttgctacgca tacgccggtt taca                                            24


<210>    54
<211>    24
<212>    DNA
<213>    Artificial Sequence

<220>
<223>    Ramp23


<400>    54
ttgctacgcc ggcgcatatt taca                                            24


<210>    55
<211>    24
<212>    DNA
<213>    Artificial Sequence

<220>
<223>    RampA


<400>    55
agaggatcag ccggaggatc acga                                              24


<210>    56
<211>    24
<212>    DNA
<213>    Artificial Sequence

<220>
<223>    RampD


<400>    56
agaggatcag acggaggatc acga                                              24


<210>    57
<211>    24
<212>    DNA
<213>    Artificial Sequence

<220>
<223>    RampG


<400>    57
agaggatcag gcggaggatc acga                                              24


<210>    58
<211>    21
<212>    DNA
<213>    Artificial Sequence

<220>
<223>    RampH


<400>    58
agaggatcag gaggatcacg a                                                 21


<210>    59
<211>    45
<212>    DNA
<213>    Artificial Sequence

<220>
<223>    Rampi


<400>    59
agaggatcac tactacgcat acgcatattt acaggaggat cacga                       45


<210>    60
<211>    24
<212>    DNA

<213>    Artificial Sequence

<220>
<223>    RampK


<400>    60
agaggatcaa aaggaggatc acga                                              24


<210>    61
<211>    24
<212>    DNA
<213>    Artificial Sequence

<220>
<223>    RampN


<400>    61
agaggatcaa acggaggatc acga                                              24


<210>    62
<211>    24
<212>    DNA
<213>    Artificial Sequence

<220>
<223>    RampR


<400>    62
agaggatcac gtggaggatc acga                                              24


<210>    63
<211>    21
<212>    PRT
<213>    Artificial Sequence

<220>
<223>    Rampi-His


<400>    63
Arg Gly Ser His His His His His His Leu Leu Arg Ile Arg Ile Phe
1               5                   10                  15

Thr Gly Gly Ser Arg
            20


<210>    64
<211>    21
<212>    PRT
<213>    Artificial Sequence

<220>
<223>    Ramp5-His


<400>    64

Arg Gly Ser His His His His His His Arg Cys Arg Cys Phe Thr Phe
1               5                   10                  15

Thr Gly Gly Ser Arg
            20

<210>   65
<211>   21
<212>   PRT
<213>   Artificial Sequence

<220>
<223>   Ramp9-His

<400>   65
Arg Gly Ser His His His His His His Leu Leu Arg Cys Arg Cys Phe
1               5                   10                  15

Thr Gly Gly Ser Arg
            20

<210>   66
<211>   13
<212>   PRT
<213>   Artificial Sequence

<220>
<223>   RampH-His

<400>   66
Arg Gly Ser His His His His His His Gly Gly Ser Arg
1               5                   10

<210>   67
<211>   13
<212>   PRT
<213>   Artificial Sequence

<220>
<223>   RampD5-His

<400>   67
Arg Gly Ser Asp His His His His His Gly Gly Ser Arg
1               5                   10

<210>   68
<211>   14
<212>   PRT
<213>   Artificial Sequence

<220>
<223>   RampD6-His

<400>   68
Arg Gly Ser Asp His His His His His His Gly Gly Ser Arg

1               5                   10


<210>   69
<211>   13
<212>   PRT
<213>   Artificial Sequence

<220>
<223>   RampG5-His


<400>   69
Arg Gly Ser Gly His His His His His Gly Gly Ser Arg
1               5                   10


<210>   70
<211>   14
<212>   PRT
<213>   Artificial Sequence

<220>
<223>   RampG6-His


<400>   70
Arg Gly Ser Gly His His His His His His Gly Gly Ser Arg
1               5                   10


<210>   71
<211>   14
<212>   PRT
<213>   Artificial Sequence

<220>
<223>   RampA-His


<400>   71
Arg Gly Ser Ala His His His His His His Gly Gly Ser Arg
1               5                   10


<210>   72
<211>   14
<212>   PRT
<213>   Artificial Sequence

<220>
<223>   RampK-His


<400>   72
Arg Gly Ser Lys His His His His His His Gly Gly Ser Arg
1               5                   10


<210>   73
<211>   14
<212>   PRT
<213>   Artificial Sequence

<220>
<223>    RampN-His


<400>    73
Arg Gly Ser Asn His His His His His His Gly Gly Ser Arg
  1               5                   10


<210>    74
<211>    14
<212>    PRT
<213>    Artificial Sequence

<220>
<223>    RampR-His


<400>    74
Arg Gly Ser Arg His His His His His His Gly Gly Ser Arg
  1               5                   10


<210>    75
<211>    22
<212>    PRT
<213>    Artificial Sequence

<220>
<223>    Ramp HAK


<400>    75
Arg Gly Ser His Ala Lys Ala His Ala His Ala His Ala His Gly His
  1               5                   10                  15

Ala His Gly Gly Ser Arg
            20


<210>    76
<211>    63
<212>    DNA
<213>    Artificial Sequence

<220>
<223>    Rampi-His


<400>    76
agaggatcac atcaccatca ccatcaccta ctacgcatac gcatatttac aggaggatca         60

cga                                                                       63


<210>    77
<211>    63
<212>    DNA
<213>    Artificial Sequence

<220>
<223>    Ramp5-His

<400>    77
agaggatcac atcaccatca ccatcaccgc tgtcgctgtt ttacatttac aggaggatca        60

cga                                                                       63


<210>    78
<211>    63
<212>    DNA
<213>    Artificial Sequence

<220>
<223>    Ramp9-His


<400>    78
agaggatcac atcaccatca ccatcaccta ctacgctgtc gctgttttac aggaggatca        60

cga                                                                       63


<210>    79
<211>    39
<212>    DNA
<213>    Artificial Sequence

<220>
<223>    RampH-His


<400>    79
agaggatcac atcaccatca ccatcacgga ggatcacga                                39


<210>    80
<211>    39
<212>    DNA
<213>    Artificial Sequence

<220>
<223>    RampD5-His


<400>    80
agaggatcag accatcacca tcaccatgga ggatcacga                                39


<210>    81
<211>    42
<212>    DNA
<213>    Artificial Sequence

<220>
<223>    RampD6-His


<400>    81
agaggatcag accatcacca tcaccatcac ggaggatcac ga                            42


<210>    82

<211>    39
<212>    DNA
<213>    Artificial Sequence

<220>
<223>    RampG5-His


<400>    82
agaggatcag gccatcacca tcaccatgga ggatcacga                              39


<210>    83
<211>    42
<212>    DNA
<213>    Artificial Sequence

<220>
<223>    RampG6-His


<400>    83
agaggatcag gccatcacca tcaccatcac ggaggatcac ga                          42


<210>    84
<211>    42
<212>    DNA
<213>    Artificial Sequence

<220>
<223>    RampA-His


<400>    84
agaggatcag cccatcacca tcaccatcac ggaggatcac ga                          42


<210>    85
<211>    42
<212>    DNA
<213>    Artificial Sequence

<220>
<223>    RampK-His


<400>    85
agaggatcaa aacatcacca tcaccatcac ggaggatcac ga                          42


<210>    86
<211>    42
<212>    DNA
<213>    Artificial Sequence

<220>
<223>    RampN-His


<400>    86
agaggatcaa accatcacca tcaccatcac ggaggatcac ga                          42

<210>    87
<211>    42
<212>    DNA
<213>    Artificial Sequence

<220>
<223>    RampR-His


<400>    87
agaggatcac gtcatcacca tcaccatcac ggaggatcac ga                          42


<210>    88
<211>    66
<212>    DNA
<213>    Artificial Sequence

<220>
<223>    Ramp HAK


<400>    88
agaggatcac acgcaaaagc gcacgcccac gcacacgcgc atggacacgc tcatggagga       60

tcacga                                                                  66


<210>    89
<211>    64
<212>    PRT
<213>    Artificial Sequence

<220>
<223>    Ramp5-COMP


<400>    89
Arg Gly Ser Arg Cys Arg Cys Phe Thr Phe Thr Gly Gly Ser Arg Gly
1               5                   10                  15

Asp Leu Gly Pro Gln Met Leu Arg Glu Leu Gln Glu Thr Asn Ala Ala
            20                  25                  30

Leu Gln Asp Val Arg Asp Tyr Leu Arg Gln Leu Val Arg Glu Ile Thr
        35                  40                  45

Phe Leu Lys Asn Thr Val Met Glu Cys Asp Ala Cys Gly Met Gln Gln
    50                  55                  60




<210>    90
<211>    64
<212>    PRT
<213>    Artificial Sequence

<220>
<223>    Ramp9-COMP

<400>    90
Arg Gly Ser Leu Leu Arg Cys Arg Cys Phe Thr Gly Gly Ser Arg Gly
1               5                   10                  15

Asp Leu Gly Pro Gln Met Leu Arg Glu Leu Gln Glu Thr Asn Ala Ala
            20                  25                  30

Leu Gln Asp Val Arg Asp Tyr Leu Arg Gln Leu Val Arg Glu Ile Thr
        35                  40                  45

Phe Leu Lys Asn Thr Val Met Glu Cys Asp Ala Cys Gly Met Gln Gln
    50                  55                  60

<210>    91
<211>    64
<212>    PRT
<213>    Artificial Sequence

<220>
<223>    Ramp12-COMP

<400>    91
Arg Gly Ser Leu Leu Leu Leu Arg Thr Arg Thr Gly Gly Ser Arg Gly
1               5                   10                  15

Asp Leu Gly Pro Gln Met Leu Arg Glu Leu Gln Glu Thr Asn Ala Ala
            20                  25                  30

Leu Gln Asp Val Arg Asp Tyr Leu Arg Gln Leu Val Arg Glu Ile Thr
        35                  40                  45

Phe Leu Lys Asn Thr Val Met Glu Cys Asp Ala Cys Gly Met Gln Gln
    50                  55                  60

<210>    92
<211>    64
<212>    PRT
<213>    Artificial Sequence

<220>
<223>    Ramp19-COMP

<400>    92
Arg Gly Ser Phe Thr Leu Leu Leu Leu Arg Cys Gly Gly Ser Arg Gly
1               5                   10                  15

Asp Leu Gly Pro Gln Met Leu Arg Glu Leu Gln Glu Thr Asn Ala Ala
            20                  25                  30

Leu Gln Asp Val Arg Asp Tyr Leu Arg Gln Leu Val Arg Glu Ile Thr
        35                  40                  45

Phe Leu Lys Asn Thr Val Met Glu Cys Asp Ala Cys Gly Met Gln Gln
    50                  55                  60

<210>    93
<211>    192
<212>    DNA
<213>    Artificial Sequence

<220>
<223>    Ramp5-COMP


<400>    93
agaggatcac gctgtcgctg ttttacattt acaggaggat cacgaggaga cctgggcccg        60

cagatgctgc gtgaactgca ggaaaccaac gctgctctgc aggacgttcg tgactacctg       120

cgtcagctgg ttcgtgaaat caccttcctg aaaaacaccg ttatggaatg cgacgcttgc       180

ggtatgcagc ag                                                           192


<210>    94
<211>    192
<212>    DNA
<213>    Artificial Sequence

<220>
<223>    Ramp9-COMP


<400>    94
agaggatcat tgctacgctg tcgctgtttt acaggaggat cacgaggaga cctgggcccg        60

cagatgctgc gtgaactgca ggaaaccaac gctgctctgc aggacgttcg tgactacctg       120

cgtcagctgg ttcgtgaaat caccttcctg aaaaacaccg ttatggaatg cgacgcttgc       180

ggtatgcagc ag                                                           192


<210>    95
<211>    210
<212>    DNA
<213>    Artificial Sequence

<220>
<223>    Ramp12-COMP


<400>    95
agaggatcac atcaccatca ccatcacttg ctattgctac gcacacgcac aggaggatca        60

cgaggagacc tgggcccgca gatgctgcgt gaactgcagg aaaccaacgc tgctctgcag       120

gacgttcgtg actacctgcg tcagctggtt cgtgaaatca ccttcctgaa aaacaccgtt       180

atggaatgcg acgcttgcgg tatgcagcag                                        210


<210>    96
<211>    210
<212>    DNA

<213>    Artificial Sequence

<220>
<223>    Ramp19-COMP


<400>    96
agaggatcac atcaccatca ccatcacttt acattgctat tgctacgctg tggaggatca      60

cgaggagacc tgggcccgca gatgctgcgt gaactgcagg aaaccaacgc tgctctgcag     120

gacgttcgtg actacctgcg tcagctggtt cgtgaaatca ccttcctgaa aaacaccgtt     180

atggaatgcg acgcttgcgg tatgcagcag                                      210


<210>    97
<211>    261
<212>    DNA
<213>    Artificial Sequence

<220>
<223>    Proinsulin glargine


<400>    97
tttgtgaacc aacacctgtg cggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcggcta a                                               261


<210>    98
<211>    261
<212>    DNA
<213>    Artificial Sequence

<220>
<223>    Proinsulin aspart


<400>    98
tttgtgaatc agcatctgtg tggtagccat ctggttgaag cactgtatct ggtttgtggt      60

gaacgtggtt ttttctacac cgataaaacc cgtcgtgaag cagaagatct gcaggttggt     120

caggttgaat taggtggtgg tcctggtgca ggtagcctgc agccgctggc actggaaggt     180

agtctgcaga aacgtggtat tgttgaacag tgttgtacca gcatttgtag cctgtatcag     240

ctggaaaact attgcaacta a                                               261


<210>    99
<211>    261
<212>    DNA
<213>    Artificial Sequence

<220>
<223>    Proinsulin lispro


<400>    99
tttgttaatc agcatctgtg tggtagccat ctggttgaag cactgtatct ggtttgtggt         60

gaacgtggtt ttttctatac caaaccgaca cgtcgtgaag cagaagatct gcaggttggt        120

caggttgaat taggtggtgg tcctggtgca ggtagcctgc agccgctggc actggaaggt        180

agtctgcaga aacgtggtat tgttgaacag tgttgtacca gcatttgtag cctgtatcag        240

ctggaaaact attgcaacta a                                                  261


<210>    100
<211>    86
<212>    PRT
<213>    Artificial Sequence

<220>
<223>    Proinsulin glargine


<400>    100
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Gly
                85


<210>    101
<211>    84
<212>    PRT
<213>    Artificial Sequence

<220>
<223>    Proinsulin aspart


<400>    101
Phe Val Asn Gln His Leu Cys Gly Ser His Leu Val Glu Ala Leu Tyr
1               5                   10                  15

Leu Val Cys Gly Glu Arg Gly Phe Phe Tyr Thr Asp Lys Thr Glu Ala
            20                  25                  30

Glu Asp Leu Gln Val Gly Gln Val Glu Leu Gly Gly Gly Pro Gly Ala
        35                  40                  45

Gly Ser Leu Gln Pro Leu Ala Leu Glu Gly Ser Leu Gln Lys Arg Gly
    50                  55                  60

Ile Val Glu Gln Cys Cys Thr Ser Ile Cys Ser Leu Tyr Gln Leu Glu
65                  70                  75                  80

Asn Tyr Cys Asn

<210>    102
<211>    84
<212>    PRT
<213>    Artificial Sequence

<220>
<223>    Proinsulin lispro

<400>    102
Phe Val Asn Gln His Leu Cys Gly Ser His Leu Val Glu Ala Leu Tyr
1               5                   10                  15

Leu Val Cys Gly Glu Arg Gly Phe Phe Tyr Thr Lys Pro Thr Glu Ala
            20                  25                  30

Glu Asp Leu Gln Val Gly Gln Val Glu Leu Gly Gly Gly Pro Gly Ala
        35                  40                  45

Gly Ser Leu Gln Pro Leu Ala Leu Glu Gly Ser Leu Gln Lys Arg Gly
    50                  55                  60

Ile Val Glu Gln Cys Cys Thr Ser Ile Cys Ser Leu Tyr Gln Leu Glu
65                  70                  75                  80

Asn Tyr Cys Asn

<210>    103
<211>    86
<212>    PRT
<213>    Artificial Sequence

<220>
<223>    Proinsulin aspart

<400>    103
Phe Val Asn Gln His Leu Cys Gly Ser His Leu Val Glu Ala Leu Tyr
1               5                   10                  15

Leu Val Cys Gly Glu Arg Gly Phe Phe Tyr Thr Asp Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln

65                  70                  75                  80

Leu Glu Asn Tyr Cys Asn
                85



<210>    104
<211>    86
<212>    PRT
<213>    Artificial Sequence

<220>
<223>    Proinsulin lispro


<400>    104
Phe Val Asn Gln His Leu Cys Gly Ser His Leu Val Glu Ala Leu Tyr
1               5                   10                  15

Leu Val Cys Gly Glu Arg Gly Phe Phe Tyr Thr Lys Pro Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

## Description

### [Technical Field]

The present invention relates to a ramp tag for insulin overexpression and a method of manufacturing insulin using the same, and more particularly to a ramp tag for insulin overexpression represented by an amino acid sequence of RGSX₁GGX₂R (wherein X₁ represents 0-8 arbitrary amino acids and X₂ represents S or T) and a method of manufacturing insulin using the ramp tag.

### [Background Art]

A recombinant protein is a protein that has undergone genetic manipulation so as to enable mass production by transplanting a gene encoding a high-value-added genetic product derived from animals, plants, yeast, or bacteria into yeast or *E. coli,* which is a host in which expression is easy to realize. Mass production of recombinant proteins was first performed in 1983 by expressing human insulin genes in *E. coli* for the treatment of diabetes. Since then, various genetic recombination expression studies have been conducted in many life science fields. Because productivity influences cost, mass production of recombinant proteins is essential for a variety of applications. Therefore, with the advent of recombination technology, it has become possible to mass-produce medical proteins or industrial enzymes useful for humans, which previously had to be obtained in trace amounts from the natural state, and moreover, insulin used for the treatment of diabetes is the representative target protein that may be produced as a recombinant protein.

Since all living organisms have the same gene-based biochemical structure and function, it is possible to produce proteins in heterologous hosts through genetic recombination. Genes may be functionally divided into two major regions: a gene regulatory region (a cis-acting element and UTR) responsible for the transcription process for synthesizing mRNA from DNA and the translation process for synthesizing protein from mRNA; and an ORF (open reading frame) that is actually expressed as a protein under transcriptional and translational control. Since the regulatory region and ORF are each modular, expression is possible by inserting only the ORF region of a target gene into a vector composed of the regulatory region to complete a functional gene unit. Thus, elements that may be used as regulatory regions for a vector have been developed, and the time point of expression and the expression level may be adjusted depending on the characteristics of the desired protein. However, since gene expression efficiency is not always the same, lots of time and money are consumed to establish optimal conditions showing the highest expression level. In particular, the greatest difficulty in the production of recombinant proteins is that expression is so poor as to be undetectable during experimentation due to problems related to intrinsic protein stability or protein folding. Accordingly, the most efficient production of a recombinant protein requires not only overexpression to an extent sufficient to be detectable, but also high solubility.

In general, engineering is attempted at the vector, host cell, and ORF levels in order to increase expression efficiency. A vector is composed of transcription/translation and replication factors, and various variants are made for each factor. Recently, in addition to these typical elements, new regulatory factors have been discovered, and many attempts have been made to control various combinations thereof in order to increase expression efficiency. In this way, individual laboratories, as well as large companies such as New England Biolabs, Promega, and Life Technology, are providing new commercialized expression modules. In this procedure, a vector provided with a functional tag that helps protein folding or recovery is used to increase protein solubility or facilitate purification. This method has been tried extensively, not only because it is the most common and simple method but also because non-interference, that is, little effect on the function of the recombinant protein, is guaranteed.

The host cell is a very important factor when providing an environment that affects the expression of a recombinant protein. Since the chaperon in the host cell affects the folding and stability of the protein and also provides a transregulatory element responsible for transcription/translation, it is considered important in protein production. In particular, since the translation process is a process of converting a nucleotide sequence into an amino acid sequence, a lot of high-energy units such as amino acids, tRNAs, etc., are consumed, so restricted tRNA is paired with a genome and expressed in order to proceed in an economical way. Thereby, the ORF of the genome is formed in the tRNA pool of the host cell, and thus the codon usage of the ORF shows a bias in which 61 codons are not uniformly distributed but individual amino acids are expressed with restricted codons for each cell. This codon bias is a characteristic of all living organisms, with yeast and *E. coli* also being known to show a strong codon bias.

In the case in which the foreign ORF codon is not similar to the codon usage of the host cell, consideration of codon compatibility between the ORF and the host cell system is regarded as very important for the production of a recombinant protein because a lack of tRNA causes a decrease in expression rate. Therefore, as a measure of compatibility, CAI, tAI, etc. have been developed to evaluate the expression efficiency of ORF in the host cell system. CAI is a codon adaptation index in which the greater the number of codons constituting the overexpressed gene of the host cell in the foreign ORF, the higher the score. If there is a codon having a low CAI value, it is possible to increase the translation efficiency by substituting the same with a codon having a high CAI value. Meanwhile, tAI is a tRNA adaptation index showing the relationship between the overexpressed gene of the host cell and the tRNA pool. It is known to show a significant pattern in several bacteria and phage species, including *E. coli* K-12, and tAI is considered to improve translation efficiency, like CAI.

With the goal of solving the problem with protein expression caused by codon usage, substituting the codon of the recombinant protein with the codon of the host cell, which is called codon optimization, has been attempted. Codon optimization has recently been commercialized and is widely used because it is a simple method of replacing a rare codon with a frequent codon without changing the amino acid sequence, and greatly affects protein expression (Gupta, S, 2003; Korean Patent Application Publication No. 10-2009-0018799). Moreover, a method of improving the expression efficiency of a target protein by grafting only optimized ORF codons with optimized secretion signal codons has been reported (Korean Patent No. 10-0919704).

Codon optimization is a method of increasing the expression of a recombinant protein by maximizing gene expression in the translation step to increase the expression rate of wild-type genes. It serves to adjust compatibility so that the codons of plant-derived genes are matched to *E. coli* or so that the codons of animal-derived genes are easily expressed in plant systems. This process does not change the sequence of amino acids at all, but only substitutes the frequent codon for the rare codon in order to improve translation efficiency, which is problematic due to the rare codon. For example, in *E. coli,* UUA(75), which is a rare codon of leucine, is substituted with CUG(398).

In addition, a method of increasing expression of a heterologous protein in a host cell by including a first heterologous polynucleotide sequence and a second polynucleotide sequence encoding a selectable marker protein has been reported. Here, the second polynucleotide sequence encoding the selectable marker protein is deoptimized, which means that the polynucleotide is modified so that translation of the protein encoded by the polynucleotide is not optimal in the host cell into which the polynucleotide is introduced (Westwood, AD et al, 2010; Korean Patent Application Publication No. 10-2012-0041245).

Since the advent of databases containing entire genome sequences, codon optimization is easily performed on a computer based on the codon usage for each species of living organism. When the sequence of the target gene is input and the matching codon table of the host cell is input, results are generated in order from the highest score through substitution with the codon showing the highest frequency. However, since the DNA sequence of the original ORF is changed to another one, DNA has to be artificially synthesized to reflect the changed sequence, so there is a cost burden, which is undesirable. Furthermore, a problem in that the expression of a codon-optimized gene is not always improved commonly occurs.

Recently disclosed is an invention regarding a ramp tag capable of solving the problem of instability of a translation rate caused by poor compatibility between codons in foreign genes and host cells when expressing a recombinant protein in *E. coli.* This is based on the principle by which the efficiency of expression of the target protein is increased by allowing tRNA to be reused in a manner in which only the ramp tag is fused with the target gene or is expressed independently thereof, without changing the original codon sequence, unlike existing codon optimization or codon deoptimization methods that solve the problem of rare codons (Korean Patent No. 10-1446054).

The present inventors have made great efforts to develop a ramp tag sequence suitable for insulin overexpression with a focus on improving the production process for increasing the expression efficiency, among various improvement technologies for producing recombinant insulin, and thus have developed a ramp tag sequence that is able to increase insulin-specific expression and ascertained that, when the ramp tag is used, the expression level of insulin in recombinant cells is notably increased, thereby culminating in the present invention.

### [Disclosure]

It is an object of the present invention to provide a ramp tag for insulin overexpression during the manufacture of recombinant insulin and a method of producing insulin using the ramp tag.

In order to achieve the above and other objects, the present invention provides a ramp tag for insulin overexpression represented by the following amino acid sequence:

RGSX₁GGX₂R

(wherein, X₁ represents 0-8 arbitrary amino acids and X₂ represents S or T) .

In addition, the present invention provides a ramp tag represented by an amino acid sequence selected from the group consisting of SEQ ID NOS: 1 to 4, SEQ ID NOS: 6 to 8, SEQ ID NO: 10, SEQ ID NO: 11, SEQ ID NOS: 13 to 18, and SEQ ID NOS: 20 to 23.

In addition, the present invention provides a nucleic acid encoding the ramp tag.

In addition, the present invention provides an insulin construct comprising the nucleic acid described above; and a nucleic acid encoding insulin or an insulin precursor.

In addition, the present invention provides a recombinant vector comprising the gene construct.

In addition, the present invention provides a recombinant cell comprising the gene construct.

In addition, the present invention provides a method of manufacturing insulin or an insulin precursor comprising:
(a) expressing insulin or an insulin precursor by culturing the recombinant cell; and
(b) recovering the expressed insulin or insulin precursor.

### [Description of Drawings]

FIG. 1 schematically shows a gene construct comprising a ramp tag and a target gene in a recombinant gene expression module according to various embodiments of the present invention;
FIG. 2 schematically shows a process of cloning the gene construct, designed according to various embodiments of the present invention, into an expression vector;
FIG. 3 is SDS-PAGE images confirming the result of increased insulin expression due to the ramp tag effect in recombinant cells into which the gene construct according to various embodiments of the present invention is introduced,
FIG. 3(A) showing a control group in which a fusion protein MBP and an insulin protein are fused and expressed,
FIG. 3(B) showing a control group in which a fusion protein GST and an insulin protein are fused and expressed, and
FIG. 3(C) showing an experimental group according to an embodiment of the present invention in which a ramp tag and an insulin protein are fused and expressed;
FIG. 4 is SDS-PAGE images confirming the result of increased insulin expression due to the ramp tag effect in recombinant cells into which the gene construct according to various embodiments of the present invention is introduced,
FIG. 4(A) showing a control group in which a histidine tag composed of six histidines and an insulin protein are fused and expressed, and
FIG. 4(B) showing experimental groups according to various embodiments of the present invention in which the insulin protein is expressed by simultaneously applying a histidine tag and a ramp tag; and
FIG. 5 is SDS-PAGE images showing the expression of the insulin protein by simultaneously applying the fusion protein COMP and the ramp tag in recombinant cells into which the gene construct of the fusion protein COMP and the ramp tag according to various embodiments of the present invention is introduced.

### [Mode for Invention]

Unless otherwise defined, all technical and scientific terms used herein have the same meanings as those typically understood by those skilled in the art to which the present invention belongs. Generally, the nomenclature used herein and the test method described below are well known in the art and are typical.

In the present invention, in order to increase the expression level of insulin in the process of producing insulin using a recombinant cell, various ramp tag sequences capable of increasing the expression level of insulin when expressed along with insulin are designed, and it is experimentally confirmed that the expression of insulin is significantly improved when such a ramp tag is applied to insulin expression.

Accordingly, an aspect of the present invention pertains to a ramp tag for insulin overexpression represented by the following amino acid sequence:

RGSX₁GGX₂R

(wherein, X₁ represents 0-8 arbitrary amino acids and X₂ represents S or T).

The ramp tag may be represented by an amino acid sequence selected from the group consisting of SEQ ID NO: 5, SEQ ID NO: 9, SEQ ID NO: 12, SEQ ID NO: 19, and SEQ ID NO: 24 to SEQ ID NO: 31, but is not limited thereto.

In addition, the present invention pertains to a ramp tag represented by an amino acid sequence selected from the group consisting of SEQ ID NOS: 1 to 4, SEQ ID NOS: 6 to 8, SEQ ID NO: 10, SEQ ID NO: 11, SEQ ID NOS: 13 to 18, and SEQ ID NOS: 20 to 23.

Another aspect of the present invention pertains to a nucleic acid encoding the ramp tag.

The nucleic acid is preferably represented by a sequence of 21 to 45 nucleic acids (in which the number of nucleic acids is 3n) comprising a nucleic acid sequence of AGA GGA TCA at the 5' end and a sequence of GGA GGA TCA (or ACA) CGA at the 3' end, but is not limited thereto.

In addition, the present invention pertains to a nucleic acid encoding the ramp tag, in which the nucleic acid is represented by a nucleic acid sequence selected from the group consisting of SEQ ID NO: 22 to SEQ ID NO: 42.

Still another aspect of the present invention pertains to an insulin construct comprising the nucleic acid described above and a nucleic acid encoding insulin or an insulin precursor.

In the present invention, the nucleic acid encoding the ramp tag and the nucleic acid encoding the insulin or insulin precursor may be comprised in the gene construct such that these nucleic acids are expressed in a fused form or independently. Also, the gene construct may further comprise a nucleic acid encoding a tag other than the ramp tag and/or a nucleic acid encoding a fusion protein. In a preferred embodiment, the tag other than the ramp tag and the fusion protein may be operably linked to the N-terminus, C-terminus or an internal portion of the ramp tag, to which insulin or an insulin precursor is further operably linked, so these may be expressed in a form of insulin in which the ramp tag, the other tag, and the fusion protein are fused together. In the present invention, when the tag other than the ramp tag or the fusion protein, which performs a special function with regard to solubility, detection, or localization, is bound to the inside or outside of the ramp tag and expressed along with insulin, it is possible not only to ensure the function of the other tag or the fusion protein but also to improve the expression level of insulin.

In the present invention, the tag other than the ramp tag may be one or more selected from the group consisting of a His tag, T7 tag, S-tag, Flag-tag, HA-tag, V5 epitope, PelB, and Xpress epitope, but is not limited thereto.

Also, in the present invention, the fusion protein may be one or more selected from the group consisting of COMP, GST, MBP, NusA, CBP, GFP, Thioredoxin, Mistic, Sumo, and DSB, but is not limited thereto.

The fused form of the ramp tag that may be used in the present invention and the tag other than the ramp tag may be represented by a nucleic acid sequence selected from the group consisting of SEQ ID NOS: 76 to 88, but is not limited thereto.

The fused form of the ramp tag that may be used in the present invention and the fusion protein may be represented by a nucleic acid sequence selected from the group consisting of SEQ ID NO: 93 to SEQ ID NO: 96, but is not limited thereto.

In the present invention, the insulin precursor may be an insulin glargine precursor, an insulin aspart precursor, or an insulin lispro precursor, but is not limited thereto.

In the present invention, the insulin glargine precursor may be represented by the nucleic acid sequence of SEQ ID NO: 97, the insulin aspart precursor may be represented by the nucleic acid sequence of SEQ ID NO: 98, and the insulin lispro precursor may be represented by the nucleic acid sequence of SEQ ID NO: 99. However, in order to synthesize the amino acid sequence of the insulin glargine precursor, the insulin aspart precursor, or the insulin lispro precursor, each of the above nucleic acid sequences may be appropriately modified and applied within a range that is able to be easily modified and implemented by those skilled in the art.

Yet another aspect of the present invention pertains to a recombinant vector comprising the gene construct.

In the present invention, the gene construct may be introduced into a vector selected from the group consisting of a pQE30 vector, a pET vector, a pBAD vector, a pACYC vector, a pUC18 vector, a pUC1 vector, a pAPT vector, series vectors thereof, and other overexpression induction vectors known in the art, but the present invention is not limited thereto.

Still yet another aspect of the present invention pertains to a recombinant cell comprising the gene construct.

In the present invention, the recombinant cell is configured such that the gene construct is introduced into a host cell selected from the group consisting of *Escherichia coli,* yeast, a Chinese hamster ovary cell (CHO cell), and a human cell line. Here, the host cell is preferably selected from the group consisting of *E. coli* K-12, *Saccharomyces cerevisiae,* CHO-K1, and HEK 293t, and is more preferably *E. coli* K-12, but the scope of the present invention is not limited to the above examples.

A further aspect of the present invention pertains to a method of manufacturing insulin or an insulin precursor comprising:
(a) expressing insulin or an insulin precursor by culturing the recombinant cell; and
(b) recovering the expressed insulin or insulin precursor.

As used herein, the term "insulin precursor" refers to a single-stranded peptide comprising an insulin A-chain and an insulin B-chain, with a C-peptide therebetween, and may be used interchangeably with "proinsulin". In the present invention, the insulin precursor conceptually comprises all precursor forms such as native insulin precursors, insulin analogue precursors, and derivatives thereof.

As used herein, the term "insulin" refers to a protein that controls blood sugar in the body. Native insulin is a hormone secreted by the pancreas, and typically promotes intracellular glucose uptake and inhibits the breakdown of fat, and thus plays a role in controlling blood sugar in the body. In the present invention, insulin conceptually comprises all forms such as native insulin, insulin analogues, and derivatives thereof.

For insulin, an insulin precursor (proinsulin) having no blood sugar control function is processed into insulin having a blood sugar control function. Insulin is composed of two polypeptide chains, particularly an A-chain and a B-chain, each comprising 21 and 30 amino acid residues, which are linked by two disulfide bridges. The A-chain and B-chain of native insulin may comprise the following amino acid sequences.

A-chain:

Gly-Ile-Val-Glu-Gln-Cys-Cys-Thr-Ser-Ile-Cys-Ser-Leu-Tyr-

Gln-Leu-Glu-Asn-Tyr-Cys-Asn

B-chain:

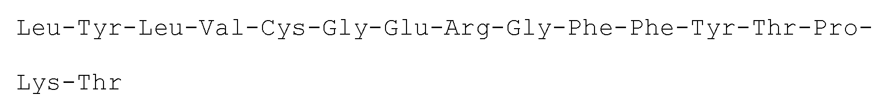

Phe-Val-Asn-Gln-His-Leu-Cys-Gly-Ser-His-Leu-Val-Glu-Ala-

The insulin precursor and insulin used in the present invention may be of human origin, but the present invention is not limited thereto.

In the present invention, the insulin analogue comprises one in which the amino acid of the B-chain or the A-chain is mutated compared to the native type. The *in-vivo* blood sugar control function of the insulin analogue may be the same as or may correspond to that of native insulin. Specifically, the insulin analogue precursor or insulin analogue may be configured such that at least one amino acid of native insulin is subjected to any variation selected from the group consisting of substitution, addition, deletion, modification, and combinations thereof, but the present invention is not limited thereto.

The insulin analogue that may be used in the present invention comprises an insulin analogue made by genetic recombination technology, and the insulin analogue conceptually comprises inverted insulin, insulin variants, insulin fragments, and the like.

The derivative has a blood sugar control function in the body, exhibits homology to each of the amino acid sequences of the A-chain and B-chain of the native insulin or insulin analogue, and comprises a peptide in a form in which some groups of one amino acid residue are chemically substituted (e.g. alpha-methylation, alpha-hydroxylation), removed (e.g. deamination), or modified (e.g. N-methylation). The insulin fragment is a form in which at least one amino acid is added to or deleted from insulin, and the added amino acid may be an amino acid that does not exist in nature (e.g. a D-type amino acid), and such an insulin fragment plays a blood sugar control function in the body.

The insulin variant is a peptide having a sequence in which at least one amino acid is different from that of insulin, and plays a blood sugar control function in the body.

The insulin analogue, derivative, fragment and variant of the present invention may be used independently or in combination. For example, a peptide, which has a sequence in which at least one amino acid is different, in which the amino-terminal amino acid residue is subjected to deamination, and which plays a blood sugar control function in the body, is also comprised in the scope of the present invention.

Specifically, in an embodiment of the present invention, the insulin analogue may be insulin glargine. Insulin glargine is stabilized by substituting asparagine, which is the 21^{st} amino acid of the A-chain of native insulin, with glycine, and is also made soluble at a weakly acidic pH by adding two arginines to the carboxy terminus of the B-chain. Here, insulin glargine is an insulin analogue developed such that it forms a microprecipitate in subcutaneous tissue when administered with an acidic solution (pH 4.0) and is slowly dissolved and released from the microprecipitate, which is an insulin glargine hexamer, whereby the action time is prolonged up to 24 hours. The A-chain and B-chain of insulin glargine may comprise the following amino acid sequences (US 5,656,722).

A-chain:

Gly-Ile-Val-Glu-Gln-Cys-Cys-Thr-Ser-Ile-Cys-Ser-Leu-Tyr-

Gln-Leu-Glu-Asn-Tyr-Cys-Gly

B-chain:

Phe-Val-Asn-Gln-His-Leu-Cys-Gly-Ser-His-Leu-Val-Glu-Ala-

Leu-Tyr-Leu-Val-Cys-Gly-Glu-Arg-Gly-Phe-Phe-Tyr-Thr-Pro-

Lys-Thr-Arg-Arg

In another embodiment, the insulin analogue in the present invention may be insulin aspart.

Insulin aspart is an insulin analogue developed such that proline, which is the 28^{th} amino acid of the B-chain of native insulin, is substituted with aspartic acid to thus reduce the tendency of insulin molecules to self-associate in a hexamer form due to the repulsive force of the charge, so it is absorbed and acts faster than native insulin. The A-chain and B-chain of insulin aspart may comprise the following amino acid sequences (US 5618913).

A-chain:

Gly-Ile-Val-Glu-Gln-Cys-Cys-Thr-Ser-Ile-Cys-Ser-Leu-Tyr-

Gln-Leu-Glu-Asn-Tyr-Cys-Asn

B-chain:

Phe-Val-Asn-Gln-His-Leu-Cys-Gly-Ser-His-Leu-Val-Glu-Ala-

Leu-Tyr-Leu-Val-Cys-Gly-Glu-Arg-Gly-Phe-Phe-Tyr-Thr-Asp-

Lys-Thr

In another embodiment, the insulin analogue in the present invention may be insulin lispro.

Insulin lispro is an insulin analogue developed such that the positions of proline, which is the 28^{th} amino acid, and lysine, which is the 29^{th} amino acid, of the B-chain of native insulin, are exchanged to thus place lysine at the 28^{th} amino acid and proline at the 29^{th} amino acid, thereby increasing the uptake rate in the body. The A-chain and B-chain of insulin lispro may comprise the following amino acid sequences (EP 0383472).

A-chain:

Gly-Ile-Val-Glu-Gln-Cys-Cys-Thr-Ser-Ile-Cys-Ser-Leu-Tyr-

Gln-Leu-Glu-Asn-Tyr-Cys-Asn

B-chain:

Phe-Val-Asn-Gln-His-Leu-Cys-Gly-Ser-His-Leu-Val-Glu-Ala-

Leu-Tyr-Leu-Val-Cys-Gly-Glu-Arg-Gly-Phe-Phe-Tyr-Thr-Lys-

Pro-Thr

The precursor of the insulin analogue described above may be represented by the following nucleic acid and amino acid sequences.

Nucleic acid sequence of insulin glargine precursor (SEQ ID NO: 97)

TTTGTGAACC AACACCTGTG CGGCTCACAC CTGGTGGAAG
CTCTCTACCT AGTGTGCGGG GAACGAGGCT TCTTCTACAC ACCCAAGACC
CGCCGGGAGG CAGAGGACCT GCAGGTGGGG CAGGTGGAGC TGGGCGGGGG
CCCTGGTGCA GGCAGCCTGC AGCCCTTGGC CCTGGAGGGG TCCCTGCAGA
AGCGTGGCAT TGTGGAACAA TGCTGTACCA GCATCTGCTC CCTCTACCAG
CTGGAGAACT ACTGCGGCTA A

Nucleic acid sequence of insulin aspart precursor (SEQ ID NO: 98)

TTTGTGAATC AGCATCTGTG TGGTAGCCAT CTGGTTGAAG

CACTGTATCT GGTTTGTGGT GAACGTGGTT TTTTCTACAC CGATAAAACC
GAAG CAGAAGATCT GCAGGTTGGT CAGGTTGAAT TAGGTGGTGG
TCCTGGTGCA GGTAGCCTGC AGCCGCTGGC ACTGGAAGGT AGTCTGCAGA
AACGTGGTAT TGTTGAACAG TGTTGTACCA GCATTTGTAG CCTGTATCAG
CTGGAAAACT ATTGCAACTA A

Nucleic acid sequence of insulin lispro precursor (SEQ ID NO: 99)

TTTGTTAATC AGCATCTGTG TGGTAGCCAT CTGGTTGAAG
CACTGTATCT GGTTTGTGGT GAACGTGGTT TTTTCTATAC CAAACCGACA
GAAG CAGAAGATCT GCAGGTTGGT CAGGTTGAAT TAGGTGGTGG
TCCTGGTGCA GGTAGCCTGC AGCCGCTGGC ACTGGAAGGT AGTCTGCAGA
AACGTGGTAT TGTTGAACAG TGTTGTACCA GCATTTGTAG CCTGTATCAG
CTGGAAAACT ATTGCAACTA A

Amino acid sequence of insulin glargine precursor (SEQ ID NO: 100)

FVNQHLCGSH LVEALYLVCG ERGFFYTPKT RREAEDLQVG
QVELGGGPGA GSLQPLALEG
SLQKRGIVEQ CCTSICSLYQ LENYCG

Amino acid sequence of insulin aspart precursor (SEQ ID NO: 101)

FVNQHLCGSH LVEALYLVCG ERGFFYTDKT EAEDLQVG QVELGGGPGA

GSLQPLALEG

SLQKRGIVEQ CCTSICSLYQ LENYCN

Amino acid sequence of insulin lispro precursor (SEQ ID NO: 102)

FVNQHLCGSH LVEALYLVCG ERGFFYTKPT EAEDLQVG QVELGGGPGA

GSLQPLALEG

SLQKRGIVEQ CCTSICSLYQ LENYCN

Amino acid sequence of insulin aspart precursor (SEQ ID NO: 103)

FVNQHLCGSH LVEALYLVCG ERGFFYTDKT RREAEDLQVG

QVELGGGPGA GSLQPLALEG 60

SLQKRGIVEQ CCTSICSLYQ LENYCN

Amino acid sequence of insulin lispro precursor (SEQ ID NO: 104)

FVNQHLCGSH LVEALYLVCG ERGFFYTKPT RREAEDLQVG
QVELGGGPGA GSLQPLALEG 60
SLQKRGIVEQ CCTSICSLYQ LENYCN

For reference, all of insulin glargine, aspart, and lispro may have the amino acid sequence of RR between the B-chain and the C-chain in the precursor state, and may show activity as short-acting insulin when the RR sequence belongs to the C-chain and is removed, depending on the type of cleavage enzyme that is used, and may act as long-acting insulin when the RR sequence remains in the B-chain. Accordingly, SEQ ID NO: 101 and SEQ ID NO: 102 may use a sequence in a form in which Arg-Arg is added between the B-chain and the C-chain.

As used herein, the term "ramp tag" refers to a synthetic tag that is able to solve the problem of instability of a translation rate caused by poor compatibility between the codon in a foreign gene and a host when expressing a recombinant protein in a recombinant cell, and is based on the principle by which the expression level of a protein is regulated only by allowing the tag to behave together inside/outside of the target gene in the transcription or translation step (inducing adoption of tRNA and providing reuse thereof) without changing the original codon sequence, unlike the existing codon optimization method that solves the problem of rare codons. A detailed description of the ramp tag in the present invention may comprise the contents set forth in Korean Patent No. 10-1446054.

As used herein, the term "gene construct" refers to a structure comprising at least one nucleic acid sequence encoding a polypeptide. In an embodiment, there is provided a gene construct comprising a nucleic acid encoding the ramp tag; and a nucleic acid encoding a tag other than the ramp tag and/or a nucleic acid encoding a fusion protein, and in another embodiment, there is provided a gene construct comprising a nucleic acid encoding the ramp tag; and a nucleic acid encoding insulin, and in still another embodiment, there is provided a gene construct comprising a nucleic acid encoding the ramp tag; a nucleic acid encoding a tag other than the ramp tag and/or a nucleic acid encoding a fusion protein; and a nucleic acid encoding insulin, in which a nucleic acid encoding a polypeptide having other functions may be further comprised, as necessary. Here, these nucleic acids are preferably operably linked so as to be expressed by one promoter, but the nucleic acid encoding the ramp tag may be located so as to be separately translated by a separate promoter.

As used herein, the term "operably linked" refers to a functional linkage between a nucleic acid expression control sequence (e.g. a promoter, a signal sequence, or a series of transcription-factor-binding sites) and a heterologous nucleic acid sequence. Here, the expression control sequence affects the transcription and/or translation of a nucleic acid corresponding to the heterologous nucleic acid sequence.

As used herein, the term "recombinant" refers to any material (e.g. a vector or microorganism) that may be formed as a result of genetic manipulation.

As used herein, the term "vector" refers to a DNA product containing a DNA sequence that is operably linked to a suitable control sequence capable of expressing DNA in a suitable host. The vector may be a plasmid, a phage particle, or a simple potential genomic insert. When transformed into an appropriate host, the vector may replicate and function independently of the host genome, or in some cases may be integrated into the genome itself. Since a plasmid is currently the most commonly used form of the vector, the terms "plasmid" and "vector" are sometimes used interchangeably in the specification of the present invention. For the purposes of the present invention, it is preferred to use a plasmid vector. A typical plasmid vector that may be used for this purpose is configured to comprise (a) a replication origin that allows efficient replication to comprise ones to hundreds of plasmid vectors in each host cell, (b) an antibiotic resistance gene that enables selection of a host cell transformed with a plasmid vector, and (c) a restriction enzyme cleavage site into which a foreign DNA fragment may be inserted. Even when an appropriate restriction enzyme cleavage site does not exist, the vector and foreign DNA may be easily ligated using a synthetic oligonucleotide adapter or linker according to a typical method. After ligation, the vector has to be transformed into an appropriate host cell. Transformation may be easily accomplished using a calcium chloride (CaCl₂) process or an electroporation process (Neumann, et al., EMBO J., 1:841, 1982).

As the vector that is used for overexpression of the gene according to the present invention, an expression vector known in the art may be used. A base sequence is said to be "operably linked" when placed in a functional relationship with another nucleic acid sequence. This may be a gene and control sequence (s) linked in such a way as to enable gene expression when an appropriate molecule (e.g. a transcriptional activation protein) is bound to the control sequence(s). For example, DNA for a pre-sequence or secretory leader is operably linked to DNA for a polypeptide when expressed as a preprotein that participates in the secretion of the polypeptide, a promoter or enhancer is operably linked to a coding sequence when it affects the transcription of the sequence, a ribosome-binding site is operably linked to a coding sequence when it affects transcription of the sequence, or a ribosome-binding site is operably linked to a coding sequence when placed to facilitate translation. In general, "operably linked" means that the linked DNA sequence is in contact therewith, or that a secretory leader is in contact therewith and is present in the reading frame. However, the enhancer need not be in contact therewith. The linking of these sequences is accomplished by ligation (linkage) at a convenient restriction enzyme site. When no such site exists, a synthetic oligonucleotide adapter or linker according to a typical method is used.

As is well known in the art, in order to increase the expression level of a transformed gene in a host cell, the gene has to be operably linked to a transcriptional and translational expression control sequence that functions in the selected expression host. Preferably, the expression control sequence and the corresponding gene are comprised in a single recombinant vector that comprises both a bacterial selection marker and a replication origin.

The recombinant cell into which the gene construct described above is introduced or which is transformed by the recombinant vector constitutes another aspect of the present invention. As used herein, the term "transformation" refers to introduction of DNA into a host such that DNA becomes replicable as an extrachromosomal factor or through chromosomal integration.

Here, it is to be understood that not all vectors function equally in expressing the DNA sequence of the present invention. Likewise, not all hosts function equally for the same expression system. However, those skilled in the art will be able to make an appropriate selection from among various vectors, expression control sequences and hosts without undue experimentation and without departing from the scope of the present invention. For example, a vector may be selected in consideration of the host. This is because the vector has to be replicated in the host. The number of copies of a vector, ability to control the number of copies, and expression of another protein encoded by the vector, for example, an antibiotic marker, also have to be taken into consideration.

Moreover, the gene construct according to the present invention may be introduced into the genome of a host cell and may be present as a chromosomal factor. It will be apparent to those skilled in the art to which the present invention belongs that, even when the gene construct is inserted into the genome chromosome of a host cell, it has the same effect as when the recombinant vector is introduced into the host cell as described above.

A better understanding of the present invention may be obtained through the following examples. These examples are merely set forth to illustrate the present invention, and are not to be construed as limiting the scope of the present invention.

### Example 1. Design of ramp tag fused to insulin gene

For the configuration of a rare codon for manufacturing a ramp tag, a rare codon table of *E. coli* as shown in Table 1 below was created by choosing a method that takes into consideration codon frequency or isoacceptor tRNA, and, only for sequences that are likely to be depleted of amino acids, normal codons were mixed, or the length of tags was limited, using a translational pairing technique.

A ramp tag suitable for an insulin protein was designed as follows.
1. Creating a rare codon table depending on a host
2. Preparing the DNA sequence of an insulin gene with codons
3. Analyzing the frequency of appearance and location of codons in the rare codon table of *E. coli* on the ORF of a target gene
4. Collecting rare codons and placing the same in the order of appearance on the ORF
5. Inducing expression by substituting the 5' or 3' of the target gene or the N-terminus (1-20 codons corresponding to the tag length) of the target protein with a tag.

**[Table 1]**

| | | | | | | | | | | |
|---|---|---|---|---|---|---|---|---|---|---|
| 2 Box tRNA Set | | | | | | 6 Box tRNA Set | | | | |
| | AA | codon | Frequency | tRNA | | | AA | Codon | Frequency | tRNA |
| H | His | CAC | 0.97 | 1 | | S | Ser | UCU | 0.80 | |
| C | Cys | UGU | 0.50 | | | | | UCC | 0.86 | 2 |
| | | UGC | 0.64 | 1 | | | | UCA | 0.71 | 1 |
| | | | | | | R | Arg | CGA | 0.35 | |
| | | | | | | | | AGG | 0.11 | 1 |
| | | | | | | | | AGA | 0.20 | 1 |
| | | | | | | L | Leu | CUA | 0.39 | 1 |
| 4 Box tRNA set | | | | | | | | | | |
| | AA | codon | Frequency | tRNA | | 3 Box and other tRNA Set | | | | |
| G | GGA | GGA | 0.79 | 1 | | | AA | codon | Frequency | tRNA |
| P | Pro | CCU | 0.70 | | | I | Ile | AUA | 0.43 | |
| | | CCC | 0.55 | 1 | | | | | | |
| | | CCA | 0.84 | 1 | | | | | | |
| T | Thr | ACU | 0.89 | | | | | | | |
| | | ACA | 0.70 | 1 | | | | | | |

### Example 2. Sequence and configuration of ramp tag fused to insulin gene

In order to provide a ramp tag that induces overexpression of insulin, which is a protein that is difficult to express, for the purpose of enabling mass production in *E. coli,* the functionality thereof was demonstrated in a form fused with a target gene.

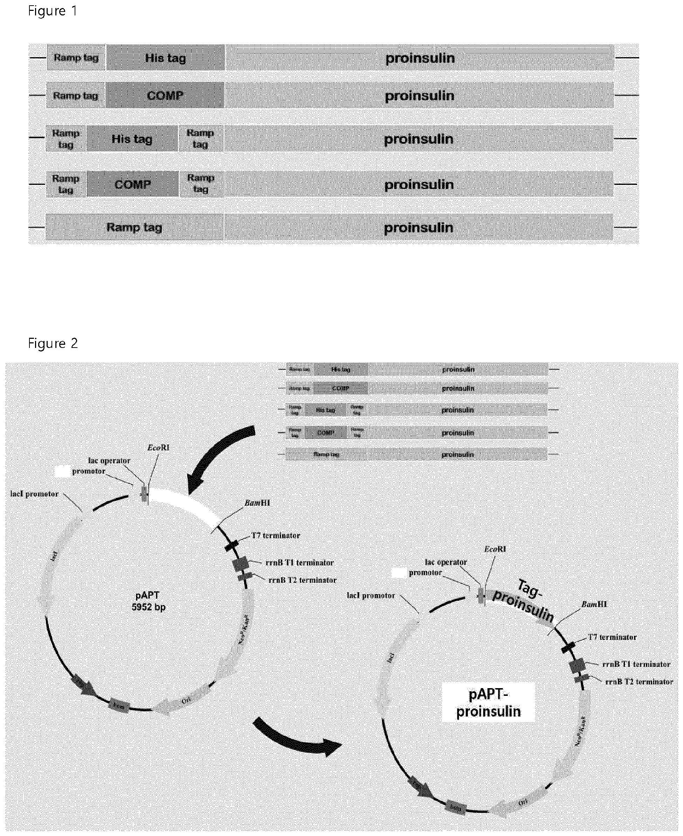

A gene construct in which a ramp tag customized to improve the low expression level of difficult-to-express proteins and the other tag were fused was designed. As shown in FIG. 1, the gene construct was designed in various forms by fusing the ramp tag alone so as to locate the same at the 5' end of a gene or by simultaneously applying the ramp tag with a separate fusion protein and locating the fusion protein that was used simultaneously therewith in the center of the ramp tag. The DNA sequence and the amino acid sequence of the insulin-gene-customized ramp tag designed in the present invention are shown in Table 2 below.

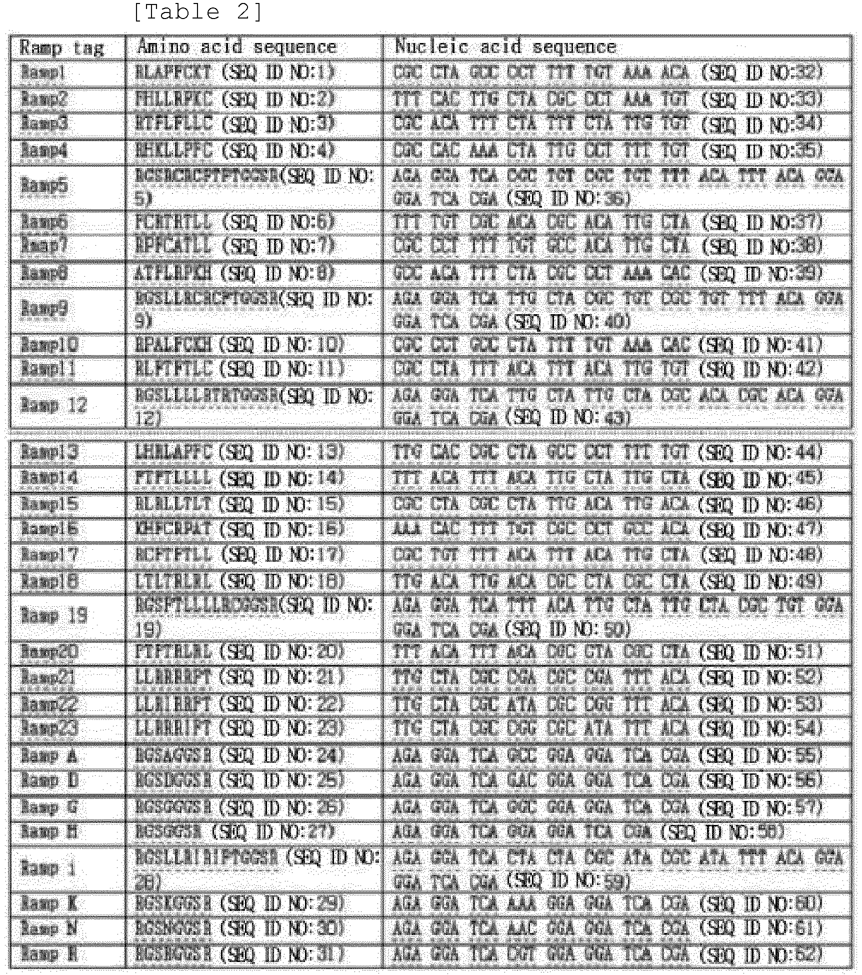

### Example 3. Analysis of expression level of insulin by fusion of ramp tag

Among the designed ramp tags in Table 2, the ramp tags of Ramp 5 and Ramp 9 were selected to induce expression in the form fused with insulin in an induction vector capable of causing a lack of tRNA. Here, the nucleic acid sequence of the ramp tag was located in front of the insulin gene to induce expression in the form fused with the insulin protein. pQE30 and pAPT were used as induction vectors for confirming the overexpression effect by the ramp tag. As a control group, a fusion protein in which MBP or GST was fused with insulin was used.

The cloning of the gene for expression of the fusion protein was carried out as shown in FIG. 2. After the gene was cloned into each vector, a forward primer in which ATG was added to the insulin-specific ramp tag sequence as shown in Table 2 was synthesized, and PCR was performed using the insulin gene of SEQ ID NO: 97 as a template, thus obtaining a gene fragment to which the ramp tag was linked. Thereafter, the restriction enzyme recognition site of the multi-cloning site was cleaved and cloned. For control groups, the size of the insulin protein comprising MBP as the fusion protein was 55.8 kDa, and the size of the insulin protein comprising GST was 33.6 kDa. The size of the insulin protein comprising the ramp tag was 10 kDa.

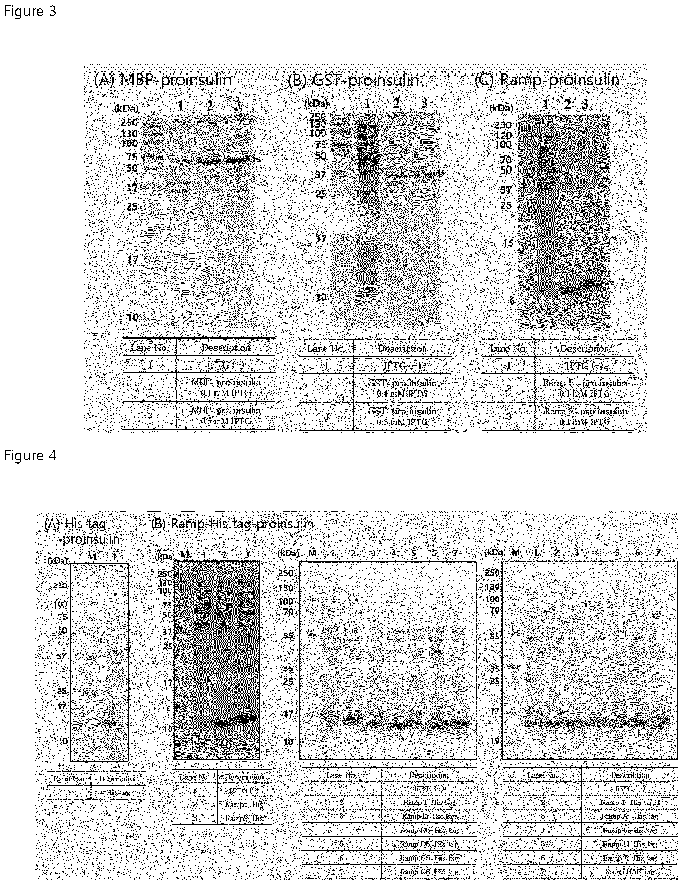

The expression levels of the proteins after addition of IPTG in the induction vector were compared using SDS-PAGE, and the results thereof are shown in FIG. 3, based on which it was confirmed that the expression level of the insulin fused with the ramp tag was increased about two times or more compared to other fusion proteins.

### Example 4. Effect of simultaneous application of histidine tag and ramp tag

Whether it is possible to induce overexpression of an insulin protein even when the ramp tag is used together with various tags for other purposes was evaluated. To this end, as shown in Table 3 below, a protein in which a histidine tag, which is commonly used as a universal tag, was fused together with the ramp tag was expressed. In addition, a complex histidine synthetic tag (histidine-alanine-lysine-(4x-alanine-histidine)-glycine-histidine-alanine-histidine (HAK)) in which histidine, alanine and lysine were used in combination was manufactured in the form of a modified histidine tag so that it was co-expressed between the ramp tags to thus express the insulin fusion protein. The size of the insulin protein comprising the histidine tag and the ramp tag was 1 kDa, and the cloning process was applied in the same manner as in Example 3.

**[Table 3]**

| Gene notation | Amino acid sequence | Base sequence |
|---|---|---|
| Ramp i-His | RGSHHHHHHLLRIRIFTGGSR (SEQ ID NO: 63) | AGA GGA TCA CAT CAC CAT CAC CAT CAC CTA CTA CGC ATA CGC ATA TTT ACA GGA GGA TCA CGA (SEQ ID NO:76) |
| Ramp 5-His | RGSHHHHHHRCRCFTFTGGSR (SEQ ID NO: 64) | AGA GGA TCA CAT CAC CAT CAC CAT CAC CGC TGT CGC TGT TTT ACA TTT ACA GGA GGA TCA CGA (SEQ ID NO: 77) |
| Ramp 9-His | RGSHHHHHHLLRCRCFTGGSR (SEQ ID NO: 65) | AGA GGA TCA CAT CAC CAT CAC CAT CAC CTA CTA CGC TGT CGC TGT TTT ACA GGA GGA TCA CGA (SEQ ID NO: 78) |
| Ramp H-His | RGSHHHHHHGGSR (SEQ ID NO: 66) | AGA GGA TCA CAT CAC CAT CAC CAT CAC GGA GGA TCA CGA (SEQ ID NO: 79) |
| Ramp D5-His | RGSDHHHHHGGSR (SEQ ID NO: 67) | AGA GGA TCA GAC CAT CAC CAT CAC CAT GGA GGA TCA |
| | | CGA (SEQ ID NO: 80) |
| Ramp D6-His | RGSDHHHHHHGGSR (SEQ ID NO: 68) | AGA GGA TCA GAC CAT CAC CAT CAC CAT CAC GGA GGA TCA CGA (SEQ ID NO: 81) |
| Ramp G5-His | RGSGHHHHHGGSR (SEQ ID NO: 69) | AGA GGA TCA GGC CAT CAC CAT CAC CAT GGA GGA TCA CGA (SEQ ID NO: 82) |
| Ramp G6-His | RGSGHHHHHHGGSR (SEQ ID NO: 70) | AGA GGA TCA GGC CAT CAC CAT CAC CAT CAC GGA GGA TCA CGA (SEQ ID NO: 83) |
| Ramp A-His | RGSAHHHHHHGGSR (SEQ ID NO: 71) | AGA GGA TCA GCC CAT CAC CAT CAC CAT CAC GGA GGA TCA CGA (SEQ ID NO: 84) |
| Ramp K-His | RGSKHHHHHHGGSR (SEQ ID NO: 72) | AGA GGA TCA AAA CAT CAC CAT CAC CAT CAC GGA GGA TCA CGA (SEQ ID NO: 85) |
| Ramp N-His | RGSNHHHHHHGGSR (SEQ ID NO: 73) | AGA GGA TCA AAC CAT CAC CAT CAC CAT CAC GGA GGA TCA CGA (SEQ ID NO: 86) |
| Ramp R-His | RGSRHHHHHHGGSR (SEQ ID NO: 74) | AGA GGA TCA CGT CAT CAC CAT CAC CAT CAC GGA GGA TCA CGA (SEQ ID NO: 87) |
| Ramp HAK | RGSHAKAHAHAHAHGHAHGGSR (SEQ ID NO: 75) | AGA GGA TCA CAC GCA AAA GCG CAC GCC CAC GCA CAC GCG CAT GGA CAC GCT CAT |
| | | GGA GGA TCA CGA (SEQ ID NO: 88) |

As shown in FIG. 4, when the expression level of the insulin protein in which the ramp tag and the other tag were fused together was compared with the expression level of insulin comprising only the histidine tag (a control group), the expression level was increased two times or more in all experimental groups to which the ramp tag and the other tag were applied together. Accordingly, it can be found that the ramp tag of the present invention was capable of exhibiting the inherent function thereof even when used in the state of being fused with a variety of other commercially available tags.

### Example 4. Effect of simultaneous application of fusion protein COMP and ramp tag

Whether it is possible to induce overexpression of an insulin protein even when the ramp tag is used together with fusion proteins for other purposes was evaluated. To this end, as shown in Table 4 below, COMP, a small fusion protein, was simultaneously applied with each of four ramp tags (Ramp 5, Ramp 9, Ramp 12, and Ramp 19) among the ramp tags shown in Table 2 to express an insulin protein. In Table 4 below, the ramp tag sequence is shown in bold text, and the COMP sequence, which is a fusion protein, is shown in plain text. The fusion protein COMP was located at the C-terminus of the ramp tag, the size of the insulin protein in which the ramp tag and COMP were fused was 18 kDa, and the cloning process was applied in the same manner as in Example 3.

**[Table 4]**

| Ramp tag | Fusion protei n | Amino acid sequence | Base sequence |
|---|---|---|---|
| Ramp 5 | COMP | RGSRCRCFTFTGGSR GDLGPQMLRELQETNAAL QD VRDYLRQLVREITFLKNT VM ECDACGMQQ (SEQ ID NO: 89) | AGAGGATCACGCTGTCGCTGTTTTACAT T TACAGGAGGATCACGA GGAGACCTGGGCCCGCAGATGCTGCGTG AA CTGCAGGAAACCAACGCTGCTCTGCAGG AC GTTCGTGACTACCTGCGTCAGCTGGTTC GT GAAATCACCTTCCTGAAAAACACCGTTA TG GAATGCGACGCTTGCGGTATGCAGCAG |
| | | | (SEQ ID NO: 93) |
| Ramp 9 | | RGSLLRCRCFTGGSR GDLGPQMLRELQETNAAL QD VRDYLRQLVREITFLKNT VM  ECDACGMQQ  (SEQ ID NO: 90) | AGAGGATCATTGCTACGCTGTCGCTGTT T TACAGGAGGATCACGA GGAGACCTGGGCCCGCAGATGCTGCGTG AA CTGCAGGAAACCAACGCTGCTCTGCAGG AC GTTCGTGACTACCTGCGTCAGCTGGTTC GT GAAATCACCTTCCTGAAAAACACCGTTA TG GAATGCGACGCTTGCGGTATGCAGCAG (SEQ ID NO: 94) |
| Ramp 12 | | RGSLLLLRTRTGGSRGDL GPQMLRELQETNAALQD VRDYLRQLVREITFLKNT VM ECDACGMQQ (SEQ ID NO: 91) | AGAGGATCATTGCTATTGCTACGCACAC GCACAGGAGGA TCACGA GGAGACCTGGGCCCGCAGATGCTGCGTG AA CTGCAGGAAACCAACGCTGCTCTGCAGG AC GTTCGTGACTACCTGCGTCAGCTGGTTC GT GAAATCACCTTCCTGAAAAACACCGTTA |
| | | | TG GAATGCGACGCTTGCGGTATGCAGCAG (SEQ ID NO: 95) |
| Ramp 19 | | RGSFTLLLLRCGGSRGDL GPQMLRELQETNAALQD VRDYLRQLVREITFLKNT VM ECDACGMQQ (SEQ ID NO: 92) | AGAGGATCATTTACATTGCTATTGCTAC GCTGTGGAGGAT CACGA GGAGACCTGGGCCCGCAGATGCTGCGTG AA CTGCAGGAAACCAACGCTGCTCTGCAGG AC GTTCGTGACTACCTGCGTCAGCTGGTTC GT GAAATCACCTTCCTGAAAAACACCGTTA TG GAATGCGACGCTTGCGGTATGCAGCAG (SEQ ID NO: 96) |

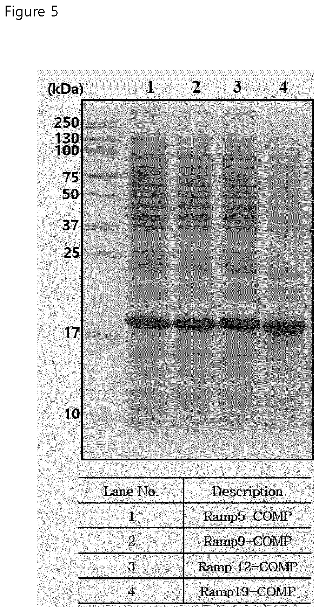

As shown in FIG. 5, when the expression level of the insulin protein in which the ramp tag and the fusion protein COMP were fused together was compared with the expression level of insulin comprising only the histidine tag (a control group), the expression level was increased two times or more in all experimental groups to which the ramp tag and COMP were applied together. Thereby, it can be found that the ramp tag of the present invention was capable of exhibiting the inherent function thereof even when used in the state of being fused with a variety of commercially available fusion proteins.

From the above description, those skilled in the art will appreciate that the present invention may be embodied in other specific forms without changing the technical spirit or essential features thereof. In this regard, the embodiments described above should be understood to be non-limiting and illustrative in every way. The scope of the present invention is defined by the claims below rather than the aforementioned detailed description, and all changes or modified forms that are capable of being derived from the meaning, range, and equivalent concepts of the appended claims should be construed as being comprised in the scope of the present invention.

### [Industrial Applicability]

When a ramp tag according to the present invention is used, in the process of manufacturing recombinant insulin, without changing the ORF sequence, such as a codon optimization method, the translation rate of insulin is increased, thereby notably increasing the expression level of insulin.

### [Sequence List Free Text]

An electronic file is attached.

## Claims

1. A ramp tag for insulin overexpression represented by a following amino acid sequence: RGSX₁GGX₂R
(wherein, X₁ represents 0-8 arbitrary amino acids and X₂ represents S or T).

2. The ramp tag according to claim 1, wherein the ramp tag is represented by an amino acid sequence selected from the group consisting of SEQ ID NO: 5, SEQ ID NO: 9, SEQ ID NO: 12, SEQ ID NO: 19, and SEQ ID NO: 24 to SEQ ID NO: 31.

3. A ramp tag represented by an amino acid sequence selected from the group consisting of SEQ ID NOS: 1 to 4, SEQ ID NOS: 6 to 8, SEQ ID NO: 10, SEQ ID NO: 11, SEQ ID NOS: 13 to 18, and SEQ ID NOS: 20 to 23.

4. A nucleic acid encoding the ramp tag according to claim 1 or 3.

5. The nucleic acid according to claim 4, wherein the nucleic acid is represented by a nucleic acid sequence selected from the group consisting of SEQ ID NO: 22 to SEQ ID NO: 42.

6. An insulin construct comprising the nucleic acid according to claim 4 and a nucleic acid encoding insulin or an insulin precursor.

7. The gene construct according to claim 6, further comprising a nucleic acid encoding a tag other than the ramp tag and/or a nucleic acid encoding a fusion protein.

8. The gene construct according to claim 7, wherein the tag other than the ramp tag is one or more selected from the group consisting of a His tag, HAK tag, T7 tag, S-tag, Flag-tag, HA-tag, V5 epitope, PelB, and Xpress epitope.

9. The gene construct according to claim 7, wherein the fusion protein is one or more selected from the group consisting of COMP, GST, MBP, NusA, CBP, GFP, Thioredoxin, Mistic, Sumo, and DSB.

10. The gene construct according to claim 6, wherein the insulin precursor is an insulin glargine precursor, an insulin aspart precursor, or an insulin lispro precursor.

11. A recombinant vector comprising the gene construct according to claim 6.

12. A recombinant cell comprising the gene construct according to claim 6.

13. The recombinant cell according to claim 12, wherein the recombinant cell is configured such that the gene construct is introduced into a host cell selected from the group consisting of *Escherichia coli,* yeast, a Chinese hamster ovary cell (CHO cell), and a human cell line.

14. A method of manufacturing insulin or an insulin precursor comprising:
(a) expressing insulin or an insulin precursor by culturing the recombinant cell according to claim 12; and
(b) recovering the expressed insulin or insulin precursor.
